(19) Europäisches Patentamt
European Patent Office
Office européen des brevets

(11) Veröffentlichungsnummer : **0 132 553**
**B1**

(12) # EUROPÄISCHE PATENTSCHRIFT

(45) Veröffentlichungstag der Patentschrift : **10.02.88**

(51) Int. Cl.[4] : **C 07 C 13/28**, C 07 C 43/184, C 09 K 19/00

(21) Anmeldenummer : **84106387.8**

(22) Anmeldetag : **05.06.84**

(54) **Bicyclohexyle.**

(30) Priorität : **14.06.83 DE 3321373**

(43) Veröffentlichungstag der Anmeldung : **13.02.85 Patentblatt 85/07**

(45) Bekanntmachung des Hinweises auf die Patenterteilung : **10.02.88 Patentblatt 88/06**

(84) Benannte Vertragsstaaten : **CH DE FR GB LI**

(56) Entgegenhaltungen :
**EP-A- 0 090 548**
**EP-A- 0 107 759**
**GB-A- 2 078 389**
**GB-A- 2 080 561**
**Patent Abstracts of Japan Band 7, Nr. 289, 23. Dezember 1983 & JP-A-58-167535**

(73) Patentinhaber : **Merck Patent Gesellschaft mit beschränkter Haftung**
**Frankfurter Strasse 250**
**D-6100 Darmstadt (DE)**

**The Secretary of State for Defence in Her Britannic Majesty's Government of the United Kingdom of Great Britain and Northern Ireland Whitehall**
**London S.W.1. (GB)**

(72) Erfinder : **Eidenschink, Rudolf, Dr.**
**Kornblumenstrasse 1**
**D-6115 Münster (DE)**
Erfinder : **Römer, Michael, Dr.**
**Im Grossen Garten 18**
**D-6094 Rodgau (DE)**
Erfinder : **Weber, Georg**
**Wilhelm-Leuschner-Strasse 38**
**D-6106 Erzhausen (DE)**
Erfinder : **Gray, George William**
**33 Newgate Street**
**Cottingham North Humberside HU16 4DY (GB)**
Erfinder : **Toyne, Kenneth Johnson, Dr.**
**25 Hall Road**
**Hull, HU6 3QW (GB)**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Jouve, 18, rue St-Denis, 75001 Paris, France

**Beschreibung**

Die Erfindung betrifft die Verwendung der Bicyclohexyle der Formel I

$$R^1—Cy—Cy—R^2 \qquad I$$

worin

$R^1$ und $R^2$ jeweils Alkyl mit 1-10 C-Atomen, worin eine oder zwei $CH_2$-Gruppe(n) durch O-Atome ersetzt sein können und

Cy 1,4-Cyclohexylen

bedeuten, als Komponenten flüssigkristalliner Dielektrika für elektrooptische Anzeigeelemente.

Diese Substanzen können wie ähnliche, z. B. aus der DE-OS 27 02 598 bekannte Verbindungen als Komponenten flüssig-kristalliner Dielektrika verwendet werden, insbesondere für Displays, die auf dem Prinzip der verdrillten Zelle, dem Guest-Host-Effekt der Deformation aufgerichteter Phasen oder dem Effekt der dynamischen Streuung beruhen.

Der Erfindung lag die Aufgabe zugrunde, neue stabile Flüssig-kristalline oder mesogene Verbindungen aufzufinden, die als Komponenten flüssig-kristalliner Dielektrika geeignet sind. Diese Aufgabe wurde durch die Bereitstellung der Verbindungen der Formel I gelöst.

In den GB-A 2 078 389 und GB-A 2 080 561 sind zwar bereits allgemeine Formeln für Komponenten flüssigkristalliner Gemische angegeben, die die Bicyclohexyle der Formel I formal umfassen ; die Flüssigkristallverbindungen aus dem Stand der Technik weisen jedoch mindestens ein Strukturelement auf, welches $\pi$-Elektronen enthält. Es wurde nun gefunden, daß die Bicyclohexyle der Formel I, die keine $\pi$-Elektronen enthaltende Strukturelemente aufweisen, vorzüglich als Komponenten flüssigkristalliner Dielektrika geeignet sind. Insbesondere sind mit ihrer Hilfe stabile flüssig-kristalline Phasen mit relativ geringer optischer Anisotropie und mit hohem nematischem Charakter herstellbar, die sich in elektrooptischen Anzeigeelementen nach dem Prinzip der verdrillten Zelle und/oder dem Guest-Host-Prinzip durch eine besonders günstige Winkelabhängigkeit des Kontrastes auszeichnen.

Mit der Bereitstellung der Verbindungen der Formel I wird außerdem ganz allgemein die Palette der flüssigkristallinen Substanzen, die sich unter verschiedenen anwendungstechnischen Gesichtspunkten zur Herstellung nematischer Gemische eignen, erheblich verbreitert.

Die Verbindungen der Formel I besitzen einen breiten Anwendungsbereich. In Abhängigkeit von der Auswahl der Substituenten können diese Verbindungen als Basismaterialien dienen, aus denen flüssigkristalline Dielektrika zum überwiegenden Teil zusammengesetzt sind ; es können aber auch Verbindungen der Formel I flüssigkristallinen Basismaterialien aus anderen Verbindungsklassen zugesetzt werden, um die optische Anisotropie oder die Winkelabhängigkeit des Kontrastes eines solchen Dielektrikums zu beeinflussen.

Die Verbindungen der Formel I sind in reinem Zustand farblos und bilden flüssigkristalline Mesophasen in einem für die elektrooptische Verwendung günstig gelegenen Temperaturbereich. Chemisch, thermisch und gegen Licht sind sie sehr stabil.

Gegenstand der Erfindung ist somit die Verwendung der Verbindungen der Formel I als Komponenten flüssigkristalliner Dielektrika, die keine $\pi$-Elektronen enthaltende Strukturelemente aufweisen. Gegenstand der Erfindung sind weiterhin flüssigkristalline Dielektrika mit einem Gehalt an mindestens einer Verbindung der Formel I sowie elektrooptische Anzeigeelemente, die derartige Dielektrika enthalten.

Vor- und nachstehend haben die Reste $R^1$, $R^2$ und Cy die angegebene Bedeutung, sofern nicht ausdrücklich etwas anderes vermerkt ist.

Gegenstand der Erfindung ist insbesondere die Verwendung der Bicyclohexyle der Formel I

worin

$R^1$ und $R^2$ jeweils Alkyl oder Alkoxy mit 1-10 C-Atomen, 3-Oxabutyl (= 2-Methoxyethyl), 3- oder 4-Oxa-pentyl, 3-, 4- oder 5-Oxahexyl, 3-, 4-, 5- oder 6-Oxaheptyl, 3-, 4-, 5-, 6- oder 7-Oxaoctyl, 3-, 4-, 5-, 6-, 7- oder 8-Oxanonyl, 3-, 4-, 5-, 6-, 7-, 8- oder 9-Oxadecyl, 1,3-Dioxabutyl (= Methoxymethoxy), 1,3-, 1,4- oder 2,4-Dioxapentyl, 1,3-, 1,4-, 1,5-, 2,4-, 2,5- oder 3,5-Dioxahexyl und

Cy 1,4-Cyclohexylen

bedeuten, als Komponenten flüssigkristalliner Dielektrika für elektrooptische Anzeigeelemente.

In den Verbindungen der Formel I sind diejenigen Stereoisomeren bevorzugt, worin die beiden 1,4-Substituenten jeweils in trans-Stellung zueinander stehen.

Die Reste $R^1$ und $R^2$ bedeuten vorzugsweise Alkyl mit 1-10, insbesondere 3, 4, 5, 6, 7 oder 8 C-Atomen, ferner auch Alkoxy oder Alkoxymethyl mit jeweils bis zu 10, vorzugsweise 2, 3, 4, 5, 6, 7 oder 8 C-Atomen. Sie sind vorzugsweise geradkettig, bedeuten also vorzugsweise Propyl, Butyl, Pentyl, Hexyl, Heptyl, Octyl, ferner Ethoxy, Propoxy, Butoxy, Pentoxy, Heptoxy, Octoxy, Methoxymethyl, Ethoxymethyl, Propoxymethyl, Butoxymethyl, Pentoxymethyl, Hexoxymethyl, Heptoxymethyl, weiterhin auch Methyl, Ethyl, Nonyl, Decyl, Methoxy, Nonoxy, Decoxy, Octoxymethyl, Nonoxymethyl, andere geradkettige Oxaalkyl- und Dioxaalkylgruppen wie 3-Oxabutyl (= 2-Methoxyethyl), 3- oder 4-Oxapentyl, 3-, 4- oder 5-Oxahexyl, 3-, 4-, 5- oder 6-Oxaheptyl, 3-, 4-, 5-, 6- oder 7-Oxaoctyl, 3-, 4-, 5-, 6-, 7- oder 8-Oxanonyl, 2-, 3-, 4-

, 5-, 6-, 7-, 8- oder 9-Oxadecyl, 1,3-Dioxabutyl (= Methoxymethoxy), 1,3-, 1,4- oder 2,4-Dioxapentyl, 1,3-, 1,4-, 1,5-, 2,4-, 2,5- oder 3,5-Dioxahexyl.

Verbindungen der Formel I mit verzweigten Flügelgruppen $R^1$ und/oder $R^2$ können gelegentlich wegen einer besseren Löslichkeit in den üblichen flüssigkristallinen Basismaterialien von Bedeutung sein, insbesondere aber als chirale Dotierstoffe, wenn sie optisch aktiv sind. Verzweigte Gruppen $R^1$ bzw. $R^2$ enthalten in der Regel nicht mehr als eine Kettenverzweigung.

Bevorzugte verzweigte Reste $R^1$ bzw. $R^2$ sind Isopropyl, 1- (sek.-Butyl) oder 2-Methyl-propyl (Isobutyl), 2-Methylbutyl, 3-Methylbutyl (Isopentyl), 2-Methylpentyl, 3-Methylpentyl, 1-Methylhexyl, 2-Ethylhexyl, 2-Propyl-pentyl, 1-Methylheptyl, Isopropoxy, 2-Methylpropoxy, 2-Methylbutoxy, 3-Methylbutoxy, 2-Methylpentoxy, 3-Methylpentoxy, 2-Ethylhexoxy, 1-Methylhexoxy, 1-Methylheptoxy, 2-Oxa-3-methylbutyl, 3-Oxa-4-methylpentyl.

Unter den Verbindungen der Formel I sind diejenigen bevorzugt, in denen mindestens einer der Reste $R^1$ bzw. $R^2$ eine der angegebenen bevorzugten Bedeutungen hat. Besonders bevorzugte Gruppen von Verbindungen entsprechen den Formeln Ia und Ib :

Alkyl—Cy—Cy—Alkyl     Ia

Alkyl—Cy—Cy—Alkoxy     Ib

worin die Alkyl- und Alkoxygruppen 1-10, vorzugsweise 2-8 C-Atome besitzen, vorzugsweise geradkettig sind und die beiden Alkylgruppen in Ia gleich oder voneinander verschieden sein können.

Die Verbindungen der Formel I werden nach an sich bekannten Methoden hergestellt, wie sie in der Literatur (z. B. in den Standardwerken wie Houben-Weyl, Methoden der Organischen Chemie, Georg-Thieme-Verlag, Stuttgart) beschrieben sind, und zwar unter Reaktionsbedingungen, die für die genannten Umsetzungen bekannt und geeignet sind. Dabei kann man auch von an sich bekannten, hier nicht näher erwähnten Varianten Gebrauch machen.

Die Ausgangsstoffe können gewünschtenfalls auch in situ gebildet werden, derart, daß man sie aus dem Reaktionsgemisch nicht isoliert, sondern sofort weiter zu den Verbindungen der Formel I umsetzt.

Als reduzierbare Gruppen kommen vorzugsweise Carbonylgruppen in Betracht, insbesondere Ketogruppen, ferner z. B. Aldehydgruppen, freie oder veresterte Hydroxygruppen. Bevorzugte Ausgangsstoffe für die Reduktion entsprechen der Formel I, enthalten aber an Stelle eines Cyclohexanrings einen Cyclohexen- oder Benzolring und/oder an Stelle einer —$CH_2$-Gruppe eine —CO-Gruppe und/oder an Stelle eines H-Atoms eine freie oder eine funktionell (z. B. eine in Form ihres p-Toluolsulfonats) abgewandelte OH-Gruppe.

Diese Ausgangsstoffe sind entweder bekannt, oder sie können in an sich bekannter Weise hergestellt werden. So sind Phenylcyclohexane des Typs $R^1$-Cy-Phe-$R^2$ (worin Phe eine 1,4-Phenylengruppe bedeutet) aus der DE-OS 26 36 684 bekannt. Cyan-bicyclohexyle des Typs $R^1$-Cy-Cy-CN sind aus der DE-OS 27 02 598 bekannt. Daraus können durch Grignard-Reaktionen Ketone des Typs $R^1$-Cy-Cy-CO-$R^3$, worin $R^3$ Alkyl mit 1-9 C-Atomen bedeutet, worin auch eine oder zwei $CH_2$-Gruppe(n) durch O-Atome ersetzt sein können, hergestellt werden. Hydrolyse der Cyan-bicyclo-hexyle liefert Carbonsäuren der Formel $R^1$-Cy-Cy-COOH, die zu Carbinolen der Formel $R^1$-Cy-Cy-$CH_2$OH reduziert werden. können. Carbinole der Formel $R^1$-Cy-Cy-CHOH-$R^3$ sind durch Reduktion der obengenannten Ketone erhältlich. Umsetzung von 4-$R^1$-cyclohexanonen mit Grignard-verbindungen der Formel $R^2$-Cy-MgBr liefert nach der Hydrolyse 1-(4-$R^2$-Cy-)-4-$R^1$-cyclohexanole, die zu 1-(4-$R^2$-Cy)-4-$R^1$-cyclohexenen dehydratisiert werden konnen.

Die Reduktion kann z. B. erfolgen durch katalytische Hydrierung bei Temperaturen zwischen etwa 0° und etwa 200° sowie Drucken zwischen etwa 1 und 200 bar in einem inerten Lösungsmittel, z. B. einem Alkohol wie Methanol, Ethanol oder Isopropanol, einem Ether wie Tetrahydrofuran (THF) oder Dioxan, einem Ester wie Ethylacetat, einer Carbonsäure wie Essigsäure oder einem Kohlenwasserstoff wie Cyclohexan. Als Katalysatoren eignen sich zweckmäßig Edelmetalle wie Pt oder Pd, die in Form von Oxiden (z. B. $PtO_2$, PdO), auf einem Träger (z. B. Pd auf Kohle, Calciumcarbonat oder Strontiumcarbonat) oder in feinverteilter Form eingesetzt werden können.

Ketone können auch nach den Methoden von Clemmensen (mit Zink, amalgamiertem Zink oder Zinn und Salzsäure, zweckmäßig in wäßrig-alkoholischer Lösung oder in heterogener Phase mit Wasser/Toluol bei Temperaturen zwischen etwa 80 und 120°) oder Wolff-Kishner (mit Hydrazin, zweckmäßig in Gegenwart von Alkali wie KOH oder NaOH in einem hochsiedenden Lösungsmittel wie Diethylenglykol oder Triethylenglykol bei Temperaturen zwischen etwa 100 und 200°) zu den entsprechenden Verbindungen der Formel I, die an Stelle der Ketogruppe eine $CH_2$-Gruppe enthalten, reduziert werden.

Weiterhin sind Reduktionen mit komplexen Hydriden möglich. Beispielsweise können Arylsulfonyloxygruppen mit $LiAlH_4$ reduktiv entfernt werden, insbesondere p-Toluol-sulfonyloxymethylgruppen zu Methylgruppen reduziert werden, zweckmäßig in einem inerten Lösungsmittel wie Diethylether oder THF bei Temperaturen zwischen etwa 0 und 100°. Doppelbindungen können mit $NaBH_4$ oder Tributylzinnnhydrid in Methanol hydriert werden.

Ether der Formel I (worin mindestens einer der Reste $R^1$ bzw. $R^2$ eine Alkylgruppe bedeutet, worin eine oder zwei $CH_2$-Gruppe(n) durch O-Atome ersetzt sind) sind durch Veretherung entsprechender

Hydroxyberbindungen erhältlich.

Entsprechende Hydroxyverbindungen sind entweder bekannt oder in an sich bekannter Weise herstellbar. So sind z. B. Bicyclohexyl-4-ole des Typs $R^1$-Cy-Cy-OH durch Reduktion entsprechender Ketone, Carbinole des Typs $R^1$-Cy-Cy-$(CH_2)_n$-OH worin n = eine ganze Zahl zwischen 1 und 9 bedeutet, durch Reduktion entsprechender Carbonsäuren erhältlich.

Zur Veretherung kann die Hydroxyverbindung zunächst in ein entsprechendes Metallderivat, z. B. durch Behandeln mit Na, K, NaH oder KH in das entsprechende Alkali-metallalkoholat umgewandelt werden. Dieses kann dann mit einem Alkylhalogenid oder -sulfonat oder einem Dialkylsulfat umgesetzt werden, zweckmäßig in Gegenwart eines inerten Lösungsmittels wie Aceton, 1,2-Dimethoxyethan, THF, Dimethylformamid oder Dimethylsulfoxid bei Temperaturen zwischen etwa 20 und 100°.

Die erfindungsgemäßen Dielektrika bestehen aus 2 bis 15, vorzugsweise 3 bis 12 Komponenten, darunter mindestens einer Verbindung der Formel I. Die anderen Bestandteile werden vorzugsweise ausgewählt aus den nematischen oder nematogenen Substanzen (insbesondere den bekannten Substanzen) aus den Klassen der Azoxybenzole, Benzylidenaniline, Biphenyle, Terphenyle, Phenyl- oder Cyclohexylbenzoate, Cyclohexan-carbonsäurephenyl- oder -cyclohexyl-ester, Phenylcyclohexane, Cyclohexylbiphenyle, Cyclohexylcyclohexane, Cyclohexylnaphtaline, 1,4-Bis-hexylbenzole, 4,4'-bis-cyclohexylbiphenyle, Phenyl- oder Cyclohexyl-pyrimidine, Phenyl- oder Cyclohexyldioxane, gegebenenfalls halogenierten Stilbene, Benzylphenylether, Tolane, substituierten Zimtsäuren, Naphthaline, Di-, Tetra- und Dekahydronaphthaline.

Die wichtigsten als Bestandteile derartiger flüssigkristalliner Dielektrika in Frage kommenden Verbindungen lassen sich durch die Formel II charakterisieren,

$$R—A—B—D—R' \qquad (II)$$

worin A und D je ein carbo- oder heterocyclisches Ringsystem aus der aus 1,4-disubstituierten Benzol- und Cyclohexanringen, 4,4'-disubstituierten Biphenyl-, Phenylcyclohexan- und Cyclohexylcyclohexansystemen, 2,5-disubstituierten Pyrimidin- und 1,3-Dioxanringen, 2,6-disubstituiertem Naphthalin, Di-, Tetra- und Dekahydronaphthalin, Chinazolin und Tetrahydrochinazolin gebildeten Gruppen,

| | |
|---|---|
| B —CH=CH— | —N(O)=N— |
| —CH=CY— | —CH=N(O)— |
| —C≡C— | $—CH_2—CH_2—$ |
| —CO—O— | $—CH_2—O—$ |
| —CO—S— | $—CH_2—S—$ |
| —CH=N— | —COO—Ph—COO— |

oder eine C-C-Einfachbindung, Y Halogen, vorzugsweise Chlor, oder —CN, und R und R' Alkyl, Alkoxy, Alkanoyloxy oder Alkoxycarbonyloxy mit bis zu 18, vorzugsweise bis zu 8 Kohlenstoffatomen, oder einer dieser Reste auch —CN, —NC, $—NO_2$, $—CF_3$, F, Cl oder Br bedeuten.

Bei den meisten dieser Verbindungen sind R und R' voneinander verschieden, wobei einer dieser Reste meist eine Alkyl- oder Alkoxygruppe ist. Aber auch andere Varianten der vorgesehenen Substituenten sind gebräuchlich. Viele solcher Substanzen oder auch Gemische davon sind im Handel erhältlich.

Die Herstellung der erfindungsgemäßen Dielektrika erfolgt in an sich üblicher Weise. In der Regel werden die Komponenten ineinander gelöst, zweckmäßig bei erhöhter Temperatur.

Durch geeignete Zusätze können die flüssigkristallinen Dielektrika nach der Erfindung so modifiziert werden, daß sie in allen bisher bekannt gewordenen Arten von Flüssigkristallanzeigeelementen verwendet werden können.

Derartige Zusätze sind dem Fachmann bekannt und in der Literatur ausführlich beschrieben. Beispielsweise können Leitsalze, vorzugsweise Ethyl-dimethyl-dodecyl-ammonium-4-hexyloxybenzoat, Tetrabutylammonium-tetraphenylboranat oder Komplexsalze von Kronenethern (vgl. z. B. I. Haller et al., Mol. Cryst. Liq. Cryst. Band 24, Seiten 249-258 (1073)) zur Verbesserung der Leitfähigkeit, dichroitische Farbstoffe oder Substanzen zur Veränderung der dielektrischen Anisotropie, der Viskosität und/oder der Orientierung der nematischen Phasen zugesetzt werden. Derartige Substanzen sind zum Beispiel in den DE-OS 22 09 127, 22 40 864, 23 21 632, 23 38 281, 24 50 088, 26 37 430, 28 53 728 und 29 02 177 beschrieben.

Die folgenden Beispiele sollen die Erfindung erläutern, ohne sie zu begrenzen. In den Beispielen bedeuten F. den Schmelzpunkt und K. den Klärpunkt einer flüssigkristallinen Substanz. Vor- und nachstehend bedeuten Prozentangaben Gewichtsprozent; alle Temperaturen sind in Grad Celsius angegeben. « Übliche Aufarbeitung » bedeutet: man gibt Wasser hinzu, extrahiert mit Dichlormethan oder Toluol, trennt ab, trocknet die organische Phase mit Natriumsulfat, dampft ein und reinigt das Produkt durch Destillation, Kristallisation und/oder Chromatographie.

## Beispiel 1

Ein Gemisch von 26,4 g trans, trans-4-Propionyl-4'-propylbicyclohexyl (erhältlich durch Reaktion von

trans, trans-4-Cyan-4'-propyl-bicyclohexyl mit $C_2H_5MgBr$ und Hydrolyse), 30 g KOH, 50 ml 85 %igem Hydrazin und 500 ml Triethylenglykol wird 1 Std. auf 120° erwärmt. Man steigert die Temperatur langsam bis zur Zersetzung des gebildeten Hydrazons, kocht nocht 4 Std., kühlt ab, arbeitet wie üblich auf und erhält trans, trans-4,4'-Dipropyl-bicyclohexyl, K. 41°.

Analog erhält man durch Wolff-Kishner-Reduktion der entsprechenden Ketone :

trans, trans-4,4'-Diethyl-bicyclohexyl
trans, trans-4-Ethyl-4'-propyl-bicyclohexyl
trans, trans-4-Ethyl-4'-butyl-bicyclohexyl
trans, trans-4-Ethyl-4'-pentyl-bicyclohexyl
trans, trans-4-Ethyl-4'-hexyl-bicyclohexyl
trans, trans-4-Ethyl-4'-heptyl-bicyclohexyl
trans, trans-4-Ethyl-4'-octyl-bicyclohexyl
trans, trans-4-Ethyl-4'-nonyl-bicyclohexyl
trans, trans-4-Ethyl-4'-decyl-bicyclohexyl
trans, trans-4-Propyl-4'-butyl-bicyclohexyl
trans, trans-4-Propyl-4'-pentyl-bicyclohexyl
trans, trans-4-Propyl-4'-hexyl-bicyclohexyl
trans, trans-4-Propyl-4'-heptyl-bicyclohexyl
trans, trans-4-Propyl-4'-octyl-bicyclohexyl
trans, trans-4-Propyl-4'-nonyl-bicyclohexyl
trans, trans-4-Propyl-4'-decyl-bicyclohexyl
trans, trans-4,4'-Dibutyl-bicyclohexyl
trans, trans-4-Butyl-4'-pentyl-bicyclohexyl
trans, trans-4-Butyl-4'-hexyl-bicyclohexyl
trans, trans-4-Butyl-4'-heptyl-bicyclohexyl
trans, trans-4-Butyl-4'-octyl-bicyclohexyl
trans, trans-4-Butyl-4'-nonyl-bicyclohexyl
trans, trans-4-Butyl-4'-decyl-bicyclohexyl
trans, trans-4-4'-Dipentyl-bicyclohexyl
trans, trans-4-Pentyl-4'-hexyl-bicyclohexyl
trans, trans-4-Pentyl-4'-heptyl-bicyclohexyl
trans, trans-4-Pentyl-4'-octyl-bicyclohexyl
trans, trans-4-Pentyl-4'-nonyl-bicyclohexyl
trans, trans-4-Pentyl-4'-decyl-bicyclohexyl
trans, trans-4,4'-Dihexyl-bicyclohexyl
trans, trans-4-Hexyl-4'-heptyl-bicyclohexyl
trans, trans-4-Hexyl-4'-octyl-bicyclohexyl
trans, trans-4-Hexyl-4'-nonyl-bicyclohexyl
trans, trans-4-Hexyl-4'-decyl-bicyclohexyl
trans, trans-4,4'-Diheptyl-bicyclohexyl
trans, trans-4-Heptyl-4'-octyl-bicyclohexyl
trans, trans-4-Heptyl-4'-nonyl-bicyclohexyl
trans, trans-4-Heptyl-4'-decyl-bicyclohexyl
trans, trans-4,4'-Dioctyl-bicyclohexyl
trans, trans-4-Octyl-4'-nonyl-bicyclohexyl
trans, trans-4-Octyl-4'-decyl-bicyclohexyl
trans, trans-4,4'-Dinonyl-bicyclohexyl
trans, trans-4-Nonyl-4'-decyl-bicyclohexyl
trans, trans-4,4'-Didecyl-bicyclohexyl.

## Beispiel 2

Man tropft unter Rühren eine Lösung von 42 g trans, trans-4-p-Tolylsulfonyloxymethyl-4'-pentyl-bicyclohexyl (erhältlich durch Hydrolyse von trans, trans-4-Cyan-4'-pentyl-bicyclohexyl zur Carbonsäure, $LiAlH_4$-Reduktion zum Carbinol und Tosylierung) in 250 ml THF zu einem Gemisch von 5 g $LiAlH_4$ in 120 ml THF und rührt noch 12 Std. bei 68°. Nach Abkühlen, Eindampfen und üblicher Aufarbeitung erhält man trans, trans-4-Methyl-4'-pentyl-bicyclohexyl, K. 40,8°.

Analog erhält man aus den entsprechenden Tosylaten :

trans, trans-4,4'-Dimethyl-bicyclohexyl
trans, trans-4-Methyl-4'-ethyl-bicyclohexyl
trans, trans-4-Methyl-4'-propyl-bicyclohexyl, F. 33°, K. 46°
trans, trans-4-Methyl-4'-butyl-bicyclohexyl
trans, trans-4-Methyl-4'-pentyl-bicyclohexyl

trans, trans-4-Methyl-4'-hexyl-bicyclohexyl
trans, trans-4-Methyl-4'-heptyl-bicyclohexyl
trans, trans-4-Methyl-4'-octyl-bicyclohexyl
trans, trans-4-Methyl-4'-nonyl-bicyclohexyl
trans, trans-4-Methyl-4'-decyl-bicyclohexyl.

## Beispiel 3

Man hydriert eine Lösung von 24,4 g trans-1-p-Propylphenyl-4-propylcyclohexan in 500 ml Ethanol an 1 g $PtO_2$ 1 Std. bei 60° und 100 bar, kühlt ab, filtriert, dampft ein und erhält 4,4'-Dipropyl-bicyclohexyl in Form eines öligen Isomerengemisches, das in 200 ml siedendem Methanol aufgenommen wird. Die erhaltene heiße Lösung wird in eine siedende Lösung von 70 g Thioharnstoff in 300 ml Methanol gegossen. Das Gemisch wird auf 0° abgekühlt, das ausgefallene Addukt abfiltriert und mit 300 ml Petrolether (Kp. 40-60°) ausgekocht. Der ungelöste Rückstand wird mit 350 ml 2 n wässriger Kalilauge 30 Min. auf 50° erwärmt. Man säuert die Lösung mit $H_2SO_4$ an und erhält nach üblicher Aufarbeitung trans, trans-4,4'-Dipropyl-bicyclohexyl, K. 41°.

Analog erhält man durch Hydrierung entsprechender Phenylcyclohexane die folgenden Verbindungen in Form von Isomerengemischen, aus denen die trans, trans-Isomeren über die Thioharnstoffverbindungen isoliert werden können :

4,4'-Diethyl-bicyclohexyl
4-Ethyl-4'-propyl-bicyclohexyl
4-Ethyl-4'-butyl-bicyclohexyl
4-Ethyl-4'-pentyl-bicyclohexyl
4-Ethyl-4'-hexyl-bicyclohexyl
4-Ethyl-4'-heptyl-bicyclohexyl
4-Ethyl-4'-octyl-bicyclohexyl
4-Ethyl-4'-nonyl-bicyclohexyl
4-Ethyl-4'-decyl-bicyclohexyl.

## Beispiel 4

Eine Lösung von 24,8 g 1-(trans-4-Propylcyclohexyl)-4-propyl-cyclohexen [erhältlich durch Reaktion von 4-Propylcyclohexanon mit 4-Propylmagnesiumbromid zu 1-(trans-4-Propylcyclohexyl)-4-propyl-cyclohexanol und Dehydratisierung] in 300 ml Cyclohexan wird an 4 g 10 %igem PdC bei 20° und 1 bar bis zum Stillstand der $H_2$-Aufnahme hydriert. Man filtriert, arbeitet analog Beispiel 3 über das Thioharnstoff-Addukt auf und erhält trans, trans-4,4'-Dipropyl-bicyclohexyl, K. 41°.

## Beispiel 5

Zu einem Gemisch von 22 g n-Butyljodid und 4,8 g NaH in 250 ml THF tropft man bei 50° unter Rühren eine Lösung von 22,4 g trans, trans-4'-Propylbicyclohexyl-4-ol in 200 ml THF und rührt noch 2 Std. bei 50°. Nach üblicher Aufarbeitung erhält man trans, trans-4-Butoxy-4'-propyl-bicyclohexyl, K. 66°.

Analog erhält man aus den entsprechenden 4'-Alkyl-bicyclohexyl-4-olen mit den entsprechenden Alkyl- bzw. Alkoxyalkyl-bromiden, -jodiden, -chloriden oder -tosylaten :

trans, trans-4-Methoxy-4'-methyl-bicyclohexyl
trans, trans-4-Methoxy-4'-ethyl-bicyclohexyl
trans, trans-4-Methoxy-4'-propyl-bicyclohexyl
trans, trans-4-Methoxy-4'-butyl-bicyclohexyl
trans, trans-4-Methoxy-4'-pentyl-bicyclohexyl
trans, trans-4-Methoxy-4'-hexyl-bicyclohexyl
trans, trans-4-Methoxy-4'-heptyl-bicyclohexyl
trans, trans-4-Methoxy-4'-octyl-bicyclohexyl
trans, trans-4-Methoxy-4'-nonyl-bicyclohexyl
trans, trans-4-Methoxy-4'-decyl-bicyclohexyl
trans, trans-4-Ethoxy-4'-methyl-bicyclohexyl
trans, trans-4-Ethoxy-4'-ethyl-bicyclohexyl
trans, trans-4-Ethoxy-4'-propyl-bicyclohexyl, F. 45°, K. 46°
trans, trans-4-Ethoxy-4'-butyl-bicyclohexyl
trans, trans-4-Ethoxy-4'-pentyl-bicyclohexyl
trans, trans-4-Ethoxy-4'-hexyl-bicyclohexyl
trans, trans-4-Ethoxy-4'-heptyl-bicyclohexyl
trans, trans-4-Ethoxy-4'-octyl-bicyclohexyl
trans, trans-4-Ethoxy-4'-nonyl-bicyclohexyl

trans, trans-4-Ethoxy-4'-decyl-bicyclohexyl
trans, trans-4-Propoxy-4'-methyl-bicyclohexyl
trans, trans-4-Propoxy-4'-ethyl-bicyclohexyl
trans, trans-4-Propoxy-4'-propyl-bicyclohexyl
trans, trans-4-Propoxy-4'-butyl-bicyclohexyl
trans, trans-4-Propoxy-4'-pentyl-bicyclohexyl
trans, trans-4-Propoxy-4'-hexyl-bicyclohexyl
trans, trans-4-Propoxy-4'-heptyl-bicyclohexyl
trans, trans-4-Propoxy-4'-octyl-bicyclohexyl
trans, trans-4-Propoxy-4'-nonyl-bicyclohexyl
trans, trans-4-Propoxy-4'-decyl-bicyclohexyl
trans, trans-4-Butoxy-4'-methyl-bicyclohexyl
trans, trans-4-Butoxy-4'-ethyl-bicyclohexyl
trans, trans-4-Butoxy-4'-butyl-bicyclohexyl
trans, trans-4-Butoxy-4'-pentyl-bicyclohexyl
trans, trans-4-Butoxy-4'-hexyl-bicyclohexyl
trans, trans-4-Butoxy-4'-heptyl-bicyclohexyl
trans, trans-4-Butoxy-4'-octyl-bicyclohexyl
trans, trans-4-Butoxy-4'-nonyl-bicyclohexyl
trans, trans-4-Butoxy-4'-decyl-bicyclohexyl
trans, trans-4-Pentoxy-4'-methyl-bicyclohexyl
trans, trans-4-Pentoxy-4'-ethyl-bicyclohexyl
trans, trans-4-Pentoxy-4'-propyl-bicyclohexyl
trans, trans-4-Pentoxy-4'-butyl-bicyclohexyl
trans, trans-4-Pentoxy-4'-pentyl-bicyclohexyl
trans, trans-4-Pentoxy-4'-hexyl-bicyclohexyl
trans, trans-4-Pentoxy-4'-heptyl-bicyclohexyl
trans, trans-4-Pentoxy-4'-octyl-bicyclohexyl
trans, trans-4-Pentoxy-4'-nonyl-bicyclohexyl
trans, trans-4-Pentoxy-4'-decyl-bicyclohexyl
trans, trans-4-Hexoxy-4'-methyl-bicyclohexyl
trans, trans-4-Hexoxy-4'-ethyl-bicyclohexyl
trans, trans-4-Hexoxy-4'-propyl-bicyclohexyl
trans, trans-4-Hexoxy-4'-butyl-bicyclohexyl
trans, trans-4-Hexoxy-4'-pentyl-bicyclohexyl
trans, trans-4-Hexoxy-4'-hexyl-bicyclohexyl
trans, trans-4-Hexoxy-4'-heptyl-bicyclohexyl
trans, trans-4-Hexoxy-4'-octyl-bicyclohexyl
trans, trans-4-Hexoxy-4'-nonyl-bicyclohexyl
trans, trans-4-Hexoxy-4'-decyl-bicyclohexyl
trans, trans-4-Heptoxy-4'-methyl-bicyclohexyl
trans, trans-4-Heptoxy-4'-ethyl-bicyclohexyl
trans, trans-4-Heptoxy-4'-propyl-bicyclohexyl
trans, trans-4-Heptoxy-4'-butyl-bicyclohexyl
trans, trans-4-Heptoxy-4'-pentyl-bicyclohexyl
trans, trans-4-Heptoxy-4'-hexyl-bicyclohexyl
trans, trans-4-Heptoxy-4'-heptyl-bicyclohexyl
trans, trans-4-Heptoxy-4'-octyl-bicyclohexyl
trans, trans-4-Heptoxy-4'-nonyl-bicyclohexyl
trans, trans-4-Heptoxy-4'-decyl-bicyclohexyl
trans, trans-4-Octoxy-4'-methyl-bicyclohexyl
trans, trans-4-Octoxy-4'-ethyl-bicyclohexyl
trans, trans-4-Octoxy-4'-propyl-bicyclohexyl
trans, trans-4-Octoxy-4'-butyl-bicyclohexyl
trans, trans-4-Octoxy-4'-pentyl-bicyclohexyl
trans, trans-4-Octoxy-4'-hexyl-bicyclohexyl
trans, trans-4-Octoxy-4'-heptyl-bicyclohexyl
trans, trans-4-Octoxy-4'-octyl-bicyclohexyl
trans, trans-4-Octoxy-4'-nonyl-bicyclohexyl
trans, trans-4-Octoxy-4'-decyl-bicyclohexyl
trans, trans-4-Nonoxy-4'-methyl-bicyclohexyl
trans, trans-4-Nonoxy-4'-ethyl-bicyclohexyl
trans, trans-4-Nonoxy-4'-propyl-bicyclohexyl
trans, trans-4-Nonoxy-4'-butyl-bicyclohexyl
trans, trans-4-Nonoxy-4'-pentyl-bicyclohexyl

trans, trans-4-Nonoxy-4'-hexyl-bicyclohexyl
trans, trans-4-Nonoxy-4'-heptyl-bicyclohexyl
trans, trans-4-Nonoxy-4'-octyl-bicyclohexyl
trans, trans-4-Nonoxy-4'-nonyl-bicyclohexyl
trans, trans-4-Nonoxy-4'-decyl-bicyclohexyl
trans, trans-4-Decoxy-4'-methyl-bicyclohexyl
trans, trans-4-Decoxy-4'-ethyl-bicyclohexyl
trans, trans-4-Decoxy-4'-propyl-bicyclohexyl
trans, trans-4-Decoxy-4'-butyl-bicyclohexyl
trans, trans-4-Decoxy-4'-pentyl-bicyclohexyl
trans, trans-4-Decoxy-4'-hexyl-bicyclohexyl
trans, trans-4-Decoxy-4'-heptyl-bicyclohexyl
trans, trans-4-Decoxy-4'-octyl-bicyclohexyl
trans, trans-4-Decoxy-4'-nonyl-bicyclohexyl
trans, trans-4-Decoxy-4'-decyl-bicyclohexyl
trans, trans-4-Methoxymethyl-4'-methyl-bicyclohexyl
trans, trans-4-Methoxymethyl-4'-ethyl-bicyclohexyl
trans, trans-4-Methoxymethyl-4'-propyl-bicyclohexyl
trans, trans-4-Methoxymethyl-4'-butyl-bicyclohexyl
trans, trans-4-Methoxymethyl-4'-pentyl-bicyclohexyl
trans, trans-4-Methoxymethyl-4'-hexyl-bicyclohexyl
trans, trans-4-Methoxymethyl-4'-heptyl-bicyclohexyl
trans, trans-4-Methoxymethyl-4'-octyl-bicyclohexyl
trans, trans-4-Methoxymethyl-4'-nonyl-bicyclohexyl
trans, trans-4-Methoxymethyl-4'-decyl-bicyclohexyl
trans, trans-4-Ethoxymethyl-4'-methyl-bicyclohexyl
trans, trans-4-Ethoxymethyl-4'-ethyl-bicyclohexyl
trans, trans-4-Ethoxymethyl-4'-propyl-bicyclohexyl
trans, trans-4-Ethoxymethyl-4'-butyl-bicyclohexyl
trans, trans-4-Ethoxymethyl-4'-pentyl-bicyclohexyl
trans, trans-4-Ethoxymethyl-4'-hexyl-bicyclohexyl
trans, trans-4-Ethoxymethyl-4'-heptyl-bicyclohexyl
trans, trans-4-Ethoxymethyl-4'-octyl-bicyclohexyl
trans, trans-4-Ethoxymethyl-4'-nonyl-bicyclohexyl
trans, trans-4-Ethoxymethyl-4'-decyl-bicyclohexyl.

Es folgen Beispiele für erfindungsgemäße Dielektrika mit einem Gehalt an mindestens einer Verbindung der Formel I :

## Beispiel A

Ein Gemisch aus
16 % trans, trans-4-Ethoxy-4'-propyl-bicyclohexyl
12 % trans, trans-4-Butoxy-4'-propyl-bicyclohexyl
17 % p-trans-4-Propylcyclohexyl-benzonitril
23 % p-trans-4-Pentylcyclohexyl-benzonitril
22 % 4-Ethyl-4'-(trans-4-pentylcyclohexyl)-biphenyl und
10 % 4-(trans-4-Pentylcyclohexyl)-4'-(trans-4-propylcyclohexyl)-biphenyl
zeigt K. 87°.

## Beispiel B

Ein Gemisch aus
16 % trans, trans-4,4'-Dipropyl-bicyclohexyl
15 % p-trans-4-Propylcyclohexyl-benzonitril
11 % p-trans-4-Butylcyclohexyl-benzonitril
21 % p-trans-4-Pentylcyclohexyl-benzonitril
4 % 4-Cyan-4'-(trans-4-pentylcyclohexyl)-biphenyl
21 % 4-Ethyl-4'-(trans-4-pentylcyclohexyl)-biphenyl und
12 % 4-(trans-4-Pentylcyclohexyl)-4'-(trans-4-propylcyclohexyl)-biphenyl
zeigt K. 88°.

**Patentansprüche** (für die Vertragsstaaten : CH, DE, GB, LI)

1. Verwendung der Bicyclohexyle der Formel I

$$R^1—Cy—Cy—R^2 \qquad I$$

worin

R1 und $R^2$ jeweils Alkyl oder Alkoxy mit 1-10 C-Atomen, 3-Oxabutyl (= 2-Methoxyethyl), 3- oder 4-Oxapentyl, 3-, 4- oder 5-Oxahexyl, 3-, 4-, 5- oder 6-Oxaheptyl, 3-, 4-, 5-, 6- oder 7-Oxaoctyl, 3-, 4-, 5-, 6-, 7-, oder 8-Oxanonyl, 3-, 4-, 5-, 6-, 7-, 8- oder 9-Oxadecyl, 1,3-Dioxabutyl (= Methoxymethoxy), 1,3-, 1,4- oder 2,4-Dioxapentyl, 1,3-, 1,4- 1,5-, 2,4-, 2,5- oder 3,5-Dioxahexyl und

Cy 1,4-Cyclohexylen

bedeuten, als Komponenten flüssigkristalliner Dielektrika für elektrooptische Anzeigeelemente.

2. Flüssigkristallines Dielektrikum für elektrooptische Anzeigeelemente mit mindestens zwei flüssigkristallinen Komponenten, dadurch gekennzeichnet, daß mindestens eine Komponente eine Verbindung der Formal I nach Anspruch 1, ist.

3. Elektrooptisches Anzeigeelement, dadurch gekennzeichnet, daß es ein Dielektrikum nach Anspruch 2 enthält.

**Patentansprüche** (für den Vertragsstaat FR)

1. Verwendung der Bicyclohexyle der Formel I

$$R^1—Cy—Cy—R^2 \qquad I$$

worin

$R^1$ und $R^2$ jeweils Alkyl mit 1-10 C-Atomen, worin eine oder zwei $CH_2$-Gruppe(n) durch 0-Atome ersetzt sein können und

Cy 1,4-Cyclohexylen

bedeuten, als Komponenten flüssigkristalliner Dielektrika für elektrooptische Anzeigeelemente.

2. Flüssigkristallines Dielektrikum für elektrooptische Anzeigeelemente mit mindestens zwei flüssigkristallinen Komponenten, dadurch gekennzeichnet, daß mindestens eine Komponente eine Verbindung der Formel I nach Anspruch 1 ist.

3. Elektrooptisches Anzeigeelement, dadurch gekennzeichnet, daß es ein Dielektrikum nach Anspruch 2 enthält.

**Claims** (for the Contracting states : CH, DE, GB, LI)

1. The use of compounds of the formula I

$$R^1—CY—CY—R^2 \qquad I$$

wherein

$R^1$ and $R^2$ are each alkyl or alkoxy with 1-10 C atoms, 3-oxabutyl, (= 2-methoxyethyl), 3- or 4-oxapentyl, 3-, 4- or 5-oxahexyl, 3-, 4-, 5- or 6-oxaheptyl, 3-, 4-, 5-, 6- or 7-oxaoctyl, 3-, 4-, 5-, 6-, 7- or 8-oxanonyl, 3-, 4-, 5-, 6-, 7-, 8- or 9-oxadecyl, 1,3-dioxabutyl (= methoxymethoxy), 1,3-, 1,4- or 2,4-dioxapentyl, 1,3-, 1,4-, 1,5-, 2,4-, 2,5- or 3,5-dioxahexyl and

Cy is 1,4-cyclohexylene,

as components of liquid-crystal dielectrics for electrooptical display elements.

2. Liquid crystal dielectric for electrooptical display elements having at least two liquid-crystal components, characterised in that at least one component is a compound of the formula I according to Claim 1.

3. Electrooptical display element characterised in that it contains a dielectric according to Claim 2.

**Claims** (for the Contracting state FR)

1. The use of compounds of the formula I

$$R^1—Cy—Cy—R^2 \qquad I$$

wherein $R^1$ and $R^2$ are each alkyl which has 1-10 C atoms and in which one or two $CH_2$ group(s) can be replaced by 0 atoms, and Cy is 1,4-cyclohexylene, as components of liquid-crystal dielectrics for electrooptical display elements.

2. Liquid-crystal dielectric for electrooptical display elements having at least two liquid-crystal

components, characterised in that at least one component is a compound of the formula I according to Claim 1.

3. Electrooptical display element characterised in that it contains a dielectric according to Claim 2.

**Revendications** (pour les Etats contractants : CH, DE, GB, LI)

1. Application des bicyclohexyles de formule I

$$R^1—Cy—Cy—R^2 \qquad I$$

où

$R^1$ et $R^2$ représentent chacun un alcoyle ou un alcoxy ayant de 1 à 10 atomes de carbone, un 3-oxabutyle (= 2-méthoxyéthyle), 3- ou 4-oxapentyle, 3-, 4- ou 5-oxahexyle, un 3-, 4-, 5- ou 6-oxaheptyle, un 3-, 4-, 5-, 6- ou 7-oxaoctyle, un 3-, 4-, 5-, 6-, 7- ou 8-oxanonyle, un 3-, 4-, 5-, 6-, 7-, 8- ou 9-oxadécyle, un 1,3-dioxabutyle (= méthoxyméthoxy), un 1,3- 1,4- ou 2,4-dioxapentyle, un 1,3-, 1,4-, 1,5-, 2,4-, 2,5- ou 3,5-dioxahexyle et

Cy représente un 1,4-cyclohexylène,

comme composants de diélectriques à cristaux liquides pour éléments d'affichage électro-optiques.

2. Diélectrique à cristaux liquides pour éléments d'affichage électro-optiques avec au moins deux composants à cristaux liquides, caractérisé en ce qu'au moins un composant est un composé de formule I selon la revendication 1.

3. Elément d'affichage électro-optique, caractérisé en ce qu'il contient un diélectrique selon la revendication 2.

**Revendications** (pour l'État contractant FR)

1. Application des bicyclohexyles de formule I

$$R^1—Cy—Cy—R^2 \qquad I$$

où

$R^1$ et $R^2$ représentent chacun un alcoyle ayant de 1 à 10 atomes de carbone, où un ou deux groupe(s) $CH_2$ peut(peuvent) être remplacé(s) par des atomes d'oxygène et

Cy représente un 1,4-cyclohexylène,

comme composants de diélectriques à cristaux liquides pour éléments d'affichage électro-optiques.

2. Diélectrique à cristaux liquides pour éléments d'affichage électro-optiques avec au moins deux composants à cristaux liquides, caractérisé en ce qu'au moins un composant est un composé de formule I selon la revendication 1.

3. Elément d'affichage électro-optique, caractérisé en ce qu'il contient un diélectrique selon la revendication 2.